## Europäisches Patentamt

## European Patent Office

## Office européen des brevets

(19)

(11) Veröffentlichungsnummer: **0 026 023**
**B1**

(12) **EUROPÄISCHE PATENTSCHRIFT**

(45) Veröffentlichungstag der Patentschrift:
**29.12.82**

(51) Int. Cl.³: **B 01 D 19/02,** C 12 M 1/08

(21) Anmeldenummer: **80200889.6**

(22) Anmeldetag: **22.09.80**

(54) Mechanischer Entschäumer für Gas-/Flüssigkeitsreaktoren.

(30) Priorität: **25.09.79 DE 2938668**

(43) Veröffentlichungstag der Anmeldung:
**01.04.81 Patentblatt 81/13**

(45) Bekanntmachung des Hinweises auf die Patenterteilung:
**29.12.82 Patentblatt 82/52**

(84) Benannte Vertragsstaaten:
**BE CH DE FR GB IT LI LU NL**

(56) Entgegenhaltungen:
**CH-A-218 637**
**DE-C-368 574**
**DE-C-388 178**
**FR-A-2 183 574**
**US-A-4 087 261**

(73) Patentinhaber: **Bergwerksverband GmbH,**
**Franz-Fischer-Weg 61, D-4300 Essen 13 (DE)**

(72) Erfinder: **Schreiber, Anselm, Walpurgisstrasse 39,**
**D-4300 Essen 1 (DE)**

(74) Vertreter: **Frühbuss, Heinrich, Dr.rer.nat.,**
**Franz-Fischer-Weg 61, D-4300 Essen 13 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Mechanischer Entschäumer für Gas-/Flüssigkeitsreaktoren

Die Erfindung betrifft einen mechanischen Entschäumer für Gas-/Flüssigkeitsreaktoren mit einem Rotor.

Zu den wichtigsten Aufgaben, die ein Gas-/Flüssigkeitsreaktor bei der z. B. aeroben Submersfermentation mit Mikroorganismen zu erfüllen hat, gehören die Erzeugung einer großen Phasengrenzfläche zwischen Gas und Biosuspension, die periodische Erneuerung der Phasengrenzfläche zur Beseitigung der Diffusionssperren und die gleichmäßige Verteilung der Mikroorganismen in der Biosuspension, so daß lokale Entmischungen und Anreicherungen vermieden werden. Der ideale Zustand einer völlig gleichmäßigen Verteilung von Gas und Biosuspension wird in den meisten Gas-/Flüssigkeitsreaktoren (Fermentern) jedoch nicht erreicht. Statt dessen bildet sich im normalen Betriebszustand eine gut mit Gas durchmischte »flüssige« Phase (Biosuspension) mit einer darüber befindlichen, schlecht durchmischten Schaumschicht. Die Schaumbildung kann so stark sein, daß der Fermenter nur zu 30—40% mit Flüssigkeit gefüllt werden kann. In der schlecht durchmischten Schaumschicht häufen sich Mikroorganismen an, so daß es zu lokalen Entmischungen kommen kann. Außerdem wird die Abtrennung des bei der Umsetzung entstehenden Abgases (z. B. $CO_2$) von der Biosuspension erschwert. Ohne besondere Vorkehrungen geht ein Großteil der Biosuspension durch Überschäumen verloren.

Die bisher bekannten mechanischen Entschäumer, oft auch Schaumabscheider genannt, sind technisch sehr aufwendig. So ist es bekannt, im Reaktorkopf vor der Abgasöffnung hochtourige konische Drehscheiben anzuordnen, durch die die Biosuspension von den Scheiben weggeschleudert und hierbei die Schaumschicht zerstört wird, worauf das Abgas entweichen kann (DE-C-2 518 082). Diese Entschäumer arbeiten nur dann zufriedenstellend, wenn die Drehscheiben wesentlich schneller als die Rührwerkswelle rotieren. Es ist deshalb ein zweites Antriebsaggregat für die hochtourigen Drehscheiben erforderlich, welches den Energieaufwand und die Störanfälligkeit erhöht. Dies fällt insbesondere bei kleinen Gas-/Flüssigkeitsreaktoren mit bis zu 20 l Inhalt mehr ins Gewicht.

Es ist ferner bekannt, das Abgas durch Ableiten der Schaumschicht in einen externen Schaumabscheider von der Biosuspension abzutrennen. Die von Schaum befreite Biosuspension wird kontinuierlich in den Gas-/Flüssigkeitsreaktor zurückgefördert (Rehm, Einführung in die industrielle Mikrobiologie, Springer-Verlag 1971, Seite 111). Diese bekannten Reaktoren haben den Nachteil, daß der Zeitraum zwischen Entstehung und Zerstörung der Schaumblasen relativ groß ist. Dadurch wird auch die Verweilzeit einzelner Teile der Biosuspension in der schlecht durchmischten Schaumschicht so groß, das lokale Entmischungen auftreten.

Es ist weiter bekannt, bei einem Entschäumer die Wirkung von konischen Drehscheiben mit einem Röhrensystem zu verknüpfen, das als Gasaustauscher wirkt (DE-A-2 719 112). Auch ein solches System ist technisch sehr aufwendig und hierdurch der Anwendungsbereich sehr eingeschränkt.

Der Erfindung liegt die Aufgabe zugrunde, einen technisch einfach herstellbaren, aber wirkungsvollen mechanischen Entschäumer zu entwickeln, bei dem verhindert wird, daß die Schaumschicht mit dem Abgas aus dem Gas-/Flüssigkeitsreaktor befördert wird, und bei dem sich ein Gleichgewicht zwischen Schaumbildung und Schaumzerstörung einstellt, wobei die Verweilzeit der einzelnen Schaumblasen so klein wie möglich bleibt.

Diese Aufgabe wird bei einem mechanischen Entschäumer der eingangs genannten Art erfindungsgemäß durch mindestens einen gekrümmten, an der Rotorwelle 1 befestigten Stab 5 mit zwei freien Enden 6, 8 gelöst, wobei von letzteren das eine Ende 6 während der Drehung der Rotorwelle 1 in Drehrichtung schaufelähnlich in die Flüssigkeit eingreift, während das andere Ende 8 im Sinne einer Flüssigkeitsschleuder über den Flüssigkeitsspiegel 9 der Flüssigkeit 7 hinausragt. Bei dem mindestens einen gekrümmten Stab 5 handelt es sich gemäß einer Weiterbildung der Erfindung vorzugsweise um einen an beiden Enden offenen rohrförmigen Hohlstab. Auf diese Weise wird bei der Betriebsdrehzahl der Rotorachse Flüssigkeit von dem eingetauchten Stabende aufgeschaufelt, am Stab entlanggeleitet und von dem aus der Flüssigkeit herausragenden Ende durch den Schaum oberhalb des Flüssigkeitsspiegels geschleudert. Dieser Effekt wird durch von dem Stab abprallende Flüssigkeitsteilchen unterstützt. Die dadurch bewirkte Schaumzerstörung wird besonders wirkungsvoll gesteigert, wenn Flüssigkeit in das eingetauchte Ende eines als rohrförmiger Hohlstab mit offenen Enden ausgebildeten Stabes gedrückt und durch den Stab hindurchgeleitet wird, so daß sie als Flüssigkeitsstrahl aus dem aus der Flüssigkeit herausragenden Ende heraustreten und durch die Schaumschicht hindurch gegen die Reaktorwand geschleudert werden kann.

Gemäß einer Weiterbildung der Erfindung kann der mindestens eine gekrümmte Stab 5 und die Rotorwelle 1 einen veränderbaren Winkel $\alpha$ bilden. Dieser Winkel beträgt vorzugsweise ca. 60°; durch seine Veränderbarkeit wird eine einfache Anpassung an die im Fermenter jeweils vorhandene Betriebssituation möglich.

Besonders wirkungsvoll erfolgt das Aufschaufeln von Flüssigkeit, wenn erfindungsgemäß der mindestens eine gekrümmte Stab 5 zu dem in die Flüssigkeit eingreifenden Ende 6 in Drehrichtung gekrümmt ist. Dabei weist erfindungsgemäß das in die Flüssigkeit eingreifende Ende 6 in Richtung einer zur Rotorwelle 1 konzentrischen Linie.

Bei Verwendung eines gekrümmten Hohlstabes ist es erfindungsgemäß möglich, seinen lichten Querschnitt zu verändern, so daß eine Anpassung an die Prozeßbedingungen und die Reaktorgröße möglich wird. Darüber hinaus können die lichten Querschnitte an den beiden Enden 6 und 8 des Hohlstabes erfindungsgemäß verschieden sein. Hierdurch können die Strömungsgeschwindigkeiten der Flüssigkeit innerhalb des Hohlstabes den Prozeßbedingungen angepaßt werden.

Die Effektivität des zuvor beschriebenen mechanischen Entschäumers kann dadurch gesteigert werden, daß mindestens zwei gekrümmte Stäbe 5a, 5b an der Rotorwelle 1 radialsymmetrisch angeordnet sind. Es ist aber auch möglich, mehrere gekrümmte Stäbe 5a, 5b erfindungsgemäß in verschiedenen Axialabständen von der Rotorachse anzubringen, wobei sich diese dann in derselben Höhe in bezug auf die Rotorachse befinden. Ebenso ist es erfindungsgemäß möglich, mehrere gekrümmte Rohre in verschiedenen Höhen an der Rotorwelle 1 anzuordnen, so daß diese verschiedene Eintauchtiefen in die Flüssigkeit besitzen.

Außerdem besteht erfindungsgemäß eine Halterung 4 für die Stäbe 5, 5a, 5b an der Rotorwelle 1 aus einer Trägerstange 41 mit Feststellschraube 42 und Haltebügeln 44, 44a, 44b mit Halteschrauben 45, 45a, 45b sowie Distanzschrauben 43, 43a, 43b. Deshalb ist sowohl eine Änderung des Stellwinkels $\alpha$ der Stäbe zur Rotorwelle als auch eine Änderung der Entfernung der eintauchenden Rohrenden von der Rotorwelle sowie eine Höhenverstellung möglich.

Es ist äußerst überraschend, daß mit dem erfindungsgemäßen Entschäumer trotz des Entstehens zusätzlicher Turbulenzen die Schaumbildung nicht weiter zunimmt. Es zeigt sich vielmehr, daß durch die rotierenden, von den Stäben ausgehenden Flüssigkeitsstrahlen eine wirkungsvolle Schaumzirkulation hervorgerufen wird, wobei die Kontaktfläche zwischen der Suspension und der Schaumschicht vergrößert, die Verweilzeit des Schaumes also kleiner wird.

Die mit der Erfindung erzielbaren Vorteile bestehen insbesondere darin, daß bei Gas-/Flüssigkeitsreaktoren, die mit Rotoren oder Turbinen betrieben werden, auf der bereits vorhandenen Rotorwelle bzw. auf dem Turbinenläufer lediglich einfach Stäbe oder Rohre montiert werden müssen, die — vom Zurechtbiegen abgesehen — keiner zusätzlichen Bearbeitung bedürfen. Durch diese Stäbe oder Rohre wird der Schaum intensiv durchgemischt, ständig mit Flüssigkeit bzw. Suspension übersprüht und kontinuierlich auf kürzestem Wege wieder in die flüssige bzw. suspendierte Phase eingemischt. Deshalb können keine lokalen Entmischungen eintreten. Es ist weiter von Vorteil, daß bei einer Steigerung der Rotor- bzw. Turbinendrehzahl, die eine Erhöhung der Schaumbildung zur Folge hat, auch die Schaumzerstörung zunimmt. Zusätzliche Steuerungsvorrichtungen entfallen deshalb.

Ein Ausführungsbeispiel der Erfindung ist in der Zeichnung anhand eines Fermenters dargestellt, der im folgenden näher beschrieben wird. Es zeigt

Fig. 1a eine Seitenansicht des Fermenters mit einem gekrümmten Stab,

Fig. 1b eine Seitenansicht des Fermenters mit zwei gekrümmten Hohlstäben,

Fig. 2a eine Aufsicht auf den Fermenter gemäß Fig. 1a,

Fig. 2b eine Aufsicht auf den Fermenter gemäß Fig. 1b,

Fig. 3 ein Wirkungsschema des Fermenters.

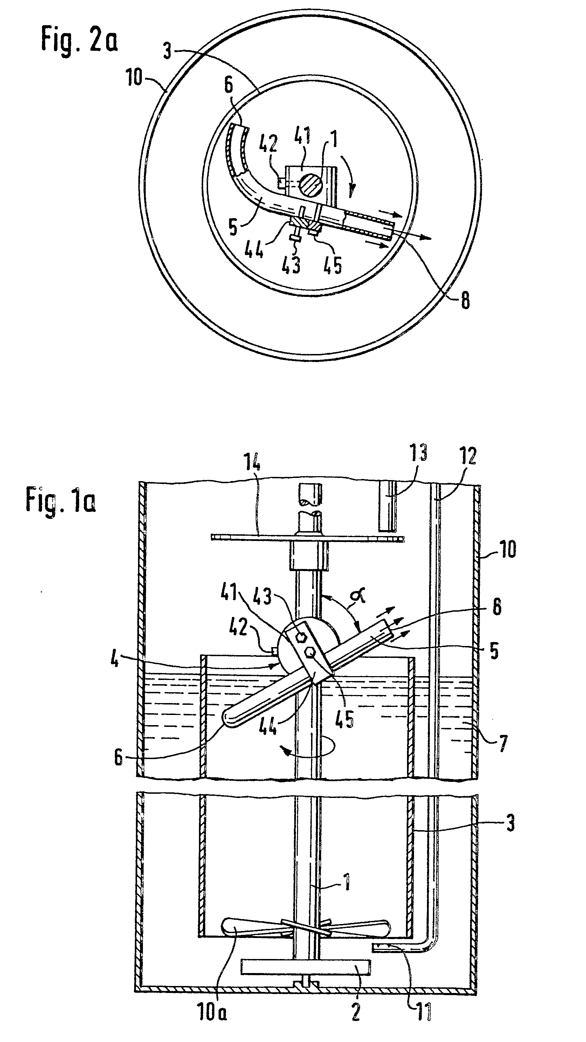

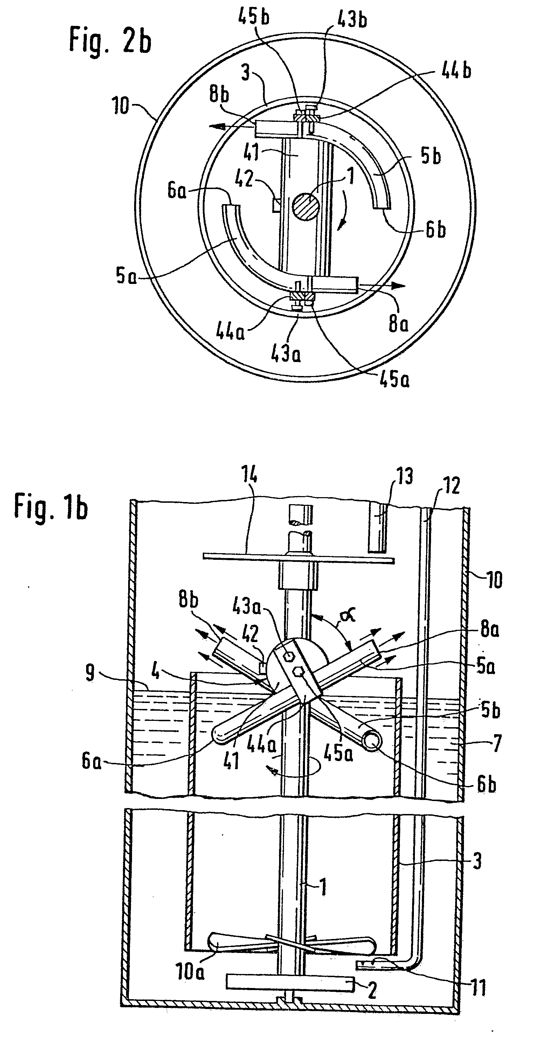

In den Fig. 1a bis 2b ist an einer vertikalen Rotorwelle 1 eines Fermenters am unteren Ende ein Magnet 2 vorgesehen. Etwa koaxial zur Rotorwelle 1 ist innerhalb des Fermenters ein an beiden Enden offener Umwurfzylinder 3 angeordnet und etwa in Höhe dessen Oberkante ein gekrümmter Stab 5 (Fig. 1a, 2a) bzw. an beiden Enden offene rohrförmige gekrümmte Hohlstäbe 5a, 5b (Fig. 1b, 2b) an der Rotorwelle 1 befestigt. Hierzu dient z. B. eine verstellbare Halterung 4.

Die Halterung 4 besteht z. B. aus einer massiven Trägerstange 41 aus Edelstahl, die eine quer zu ihrer Längsachse verlaufende Bohrung zur Aufnahme der Rotorwelle 1 aufweist und auf dieser in beliebiger Höhe mit einer Feststellschraube 42 festgestellt werden kann. Die Distanzschrauben 43 bzw. 43a und 43b dienen der mechanischen Stabilität der Haltebügel 44 bzw. 44a und 44b, die mit einer Vertiefung zur Aufnahme der Stäbe 5 bzw. an beiden Enden offenen, rohrförmigen Hohlstäbe 5a und 5b versehen sind und die es mittels der Halteschrauben 45 bzw. 45a und 45b ermöglichen, die Stäbe 5 bzw. 5a und 5b in jeder beliebigen Stellung festzuhalten. Die Stäbe 5 bzw. 5a und 5b tauchen im Betriebszustand mit ihren in die Drehrichtung gekrümmten freien Enden 6 bzw. Eintrittsöffnungen 6a und 6b in die Flüssigkeit 7 ein. Die freien Enden 8 bzw. Austrittsöffnungen 8a und 8b an den im wesentlichen geraden Endbereichen der Stäbe 5 bzw. 5a und 5b ragen über den Flüssigkeitsspiegel 9 hinaus. Der Stellwinkel $\alpha$ zwischen den Stäben 5 bzw. 5a und 5b und der Rotorwelle 1 ist veränderbar und beträgt vorzugsweise ca. 60°.

Die Rotorwelle 1 mit ihren Rotorblättern 10a am unteren freien Ende des Umwurfzylinders 3 wird von einem außerhalb des Reaktors angeordneten — hier nicht dargestellten — Magnetrührmotor über den Magneten 2 angetrieben. Bei der Drehung der Rotorwelle 1 wird Flüssigkeit von den freien Enden 6 bzw. 6a und 6b und den angrenzenden, in die Flüssigkeit 7 eingreifenden Endbereichen der Stäbe 5 bzw. 5a und 5b aufgeschaufelt, entlang den Wandungen der Stäbe 5 bzw. 5a und 5b geleitet und von den aus der Flüssigkeit 7 herausragenden freien Enden 8 bzw. 8a und 8b durch und ggf. auch über den Schaum geschleudert und bis an die Gefäßwand 10 des Reaktors gespritzt. Flüssigkeit 7, die in die Eintriffsöffnungen 6a und 6b der an beiden Enden offenen, rohrförmigen Hohlstäbe 5a und 5b gedrückt, durch sie hindurchgeleitet und durch die Austrittsöffnungen 8a und 8b an die Gefäßwand 10 gespritzt wird, verstärkt bzw. dominiert den Schaumzerstörungsprozeß; sie trifft hierbei im wesentlichen oberhalb der Schaumschicht 15 auf die Gefäßwand 10. Außerdem prallt Flüssigkeit von den eingetauchten Wandungen der Stäbe 5 bzw. 5a und 5b ab und wird durch die Schaumschicht 15 hindurch auf die Gefäßwand 10 geschleudert.

Der Fermenter wird über ein mit Düsen 11 versehenes Rohr 12 z. B. mit Luft beschickt, während die Abluft über ein Rohr 13, das durch eine Drehscheibe 14 vor hochspritzender Flüssigkeit geschützt ist, den Fermenter verläßt.

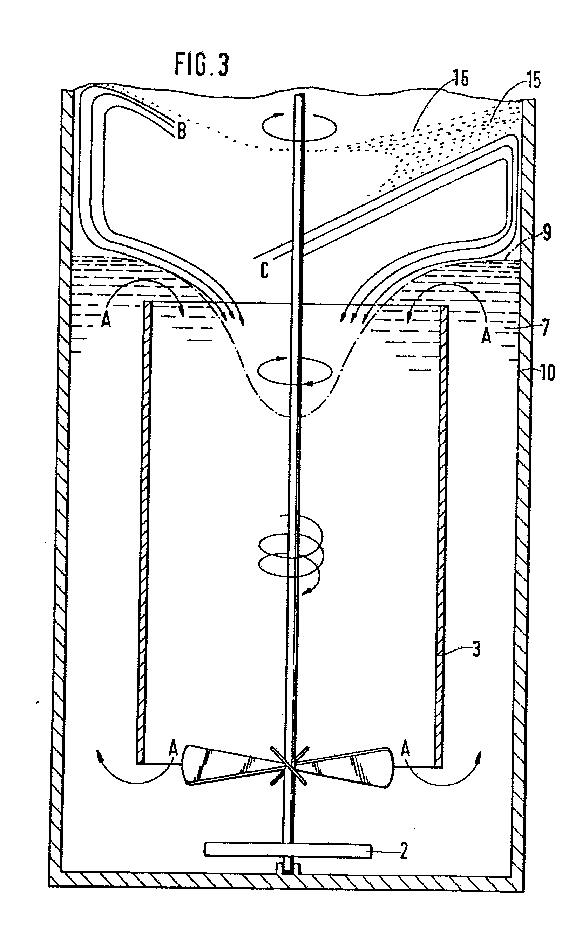

In Fig. 3 wird gezeigt, wie die Strömungen innerhalb des Fermenters verlaufen (Entschäumer selbst nicht gezeichnet). Bei der Betriebsdrehzahl zirkuliert die Flüssigkeit 7 im Umwurfzylinder 3 von oben nach unten und anschließend zwischen der Gefäßwand 10 und dem Umwurfzylinder 3 wieder nach oben (Pfeile A). Die über der Flüssigkeit 7 befindliche Schaumschicht 15 wird ohne Entschäumer nur an der Kontaktstelle mit dem Flüssigkeitsspiegel 9 in Bewegung gehalten. Erst durch die Flüssigkeitsstrahlen des Entschäumers (Pfeile B und C) wird eine Umwälzung der gesamten Schaumschicht, die bis zur Schaumgrenze 16 reicht, ein ständiger Stoffaustausch und teilweise Berieselung des Schaumes mit Flüssigkeit erreicht. Die Flüssigkeitsstrahlen (B) entstehen durch die aus den Enden 8a und 8b austretenden Flüssigkeitsstrahlen, während die mit (C) gekennzeichneten Strahlen durch das Abprallen bzw. Fortschleudern der Flüssigkeit 7 von den Stäben 5 bzw. 5a und 5b erzeugt werden. Auf diese Weise wird der Schaum in einem gegenüber herkömmlichen Entschäumern überraschend starkem Maße zerstört.

## Patentansprüche

1. Mechanischer Entschäumer für Gas-/Flüssigkeitsreaktoren mit einem Rotor, gekennzeichnet durch mindestens einen gekrümmten, an einer Rotorwelle (1) befestigten Stab (5) mit zwei freien Enden (6, 8), von denen das eine Ende (6) während der Drehung der Rotorwelle (1) in Drehrichtung schaufelähnlich in die Flüssigkeit (7) eingreift, während das andere Ende (8) im Sinne einer Flüssigkeitsschleuder über den Flüssigkeitsspiegel (9) hinausragt.

2. Entschäumer nach Anspruch 1, dadurch gekennzeichnet, daß der mindestens eine gekrümmte Stab (5) ein an beiden Enden offener rohrförmiger Hohlstab ist.

3. Entschäumer nach einem der Ansprüche 1 oder 2, dadurch gekennzeichnet, daß der mindestens eine gekrümmte Stab (5) und die Rotorwelle (1) einen veränderbaren Winkel α bilden.

4. Entschäumer nach einem oder mehreren der Ansprüche 1 bis 3, dadurch gekennzeichnet, daß der mindestens eine gekrümmte Stab (5) zu dem in die Flüssigkeit (7) eingreifenden Ende (6) hin in Drehrichtung gekrümmt ist.

5. Entschäumer nach einem oder mehreren der Ansprüche 1 bis 4, dadurch gekennzeichnet, daß das in die Flüssigkeit (7) eingreifende Ende (6) in Richtung einer zur Rotorwelle (1) konzentrischen Linie weist.

6. Entschäumer nach einem oder mehreren der Ansprüche 2 bis 5, dadurch gekennzeichnet, daß der lichte Querschnitt des mindestens einen Hohlstabes veränderbar ist.

7. Entschäumer nach einem oder mehreren der Ansprüche 2 bis 6, dadurch gekennzeichnet, daß die lichten Querschnitte an den beiden Enden (6, 8) des mindestens einen Hohlstabes verschieden sind.

8. Entschäumer nach einem oder mehreren der Ansprüche 1 bis 7, gekennzeichnet durch mindestens zwei gekrümmte Stäbe (5a, 5b), die an der Rotorwelle (1) radialsymmetrisch angeordnet sind.

9. Entschäumer nach einem oder mehreren der Ansprüche 1 bis 8, gekennzeichnet durch mehrere gekrümmte Stäbe (5a, 5b) in verschiedenen Axialabständen an der Rotorwelle (1).

10. Entschäumer nach einem oder mehreren der Ansprüche 1 bis 9, gekennzeichnet durch mehrere gekrümmte Stäbe (5) in verschiedenen Höhen an der Rotorwelle (1).

11. Entschäumer nach einem oder mehreren der Ansprüche 1 bis 10, gekennzeichnet durch eine der Befestigung des mindestens einen gekrümmten Stabes (5, 5a, 5b) an der Rotorwelle (1) dienende Halterung (4) mit einer Trägerstange (41), Feststellschraube (42) und Haltebügeln (44, 44a, 44b) mit Halteschrauben (45, 45a, 45b) sowie Distanzschrauben (43, 43a, 43b).

## Claims

1. A mechanical defoaming means for gas/liquid reactors with a rotor, characterized by at least one curved rod (5) secured to a rotor shaft (1) and having two free ends (6, 8), of which one end (6) enters the liquid (7) in the direction of rotation in the manner of a scoop as the rotor shaft (1) revolves, while the other end (8) projects above the level (9) of the liquid in the manner of a liquid centrifuge.

2. A defoaming means according to claim 1, characterized in that the at least one curved rod (5) is a tubular hollow rod open at both ends.

3. A defoaming means according to one of Claims 1 and 2, characterized in that at least one curved rod (5) and the rotor shaft (1) form a variable angle.

4. A defoaming means according to one or more of claims 1 to 3, characterized in that the at least one curved rod (5) is curved in the direction of rotation towards the end (6) entering the liquid (7).

5. A defoaming means according to one or more of claims 1 to 4, characterized in that the end (6) entering the liquid (7) points in the direction of a line concentric with the rotor shaft (1).

6. A defoaming means according to one or more of claims 2 to 5, characterized in that the inside cross-section of the at least one hollow rod is variable.

7. A defoaming means according to one or more of claims 2 to 6, characterized in that the inside cross-sections at the two ends (6, 8) of the at least one hollow rod are different.

8. A defoaming means according to one or more of claims 1 to 7, characterized by at least two curved rods (5a, 5b) which are arranged radially symmetrically on the rotor shaft (1).

9. A defoaming means according to one or more of claims 1 to 8, characterized by a plurality of curved rods (5a, 5b) at various axial distances on the rotor shaft (1).

10. A defoaming means according to one or more of claims 1 to 9, characterized by a plurality of curved rods (5) at various heights on the rotor shaft (1).

11. A defoaming means according to one or more of claims 1 to 10, characterized by a mounting (4) serving to secure the at least one curved rod (5, 5a, 5b) on the rotor shaft (1) and having a support bar (41), setscrew (42), and holding brackets (44, 44a, 44b) with holding screws (45, 45a, 45b) and distance screws (43, 43a, 43b).

## Revendications

1. Eliminateur de mousse, mécanique, pour réacteurs gaz/liquide comprenant un rotor, caractérisé par au moins une tige recourbée (5) fixée à un arbre de rotor (1) et ayant deux extrémités libres (6, 8) dont l'une des extrémités (6), pendant la rotation de l'arbre (1) du rotor, plonge dans le liquide (7) dans le sens de la rotation à la façon d'une écope tandis que l'autre (8) émerge au-dessus de la surface libre (9) du liquide à la façon d'une centrifugeuse à liquide.

2. Eliminateur de mousse suivant la revendication 1, caractérisé en ce que la tige recourbée (5) au moins au nombre de une est une tige creuse tubulaire ouverte à ses deux extrémités.

3. Eliminateur de mousse suivant l'une des revendications 1 et 2, caractérisé en ce que la tige recourbée (5) au moins au nombre de une et l'arbre (1) du rotor font un angle α variable.

4. Eliminateur de mousse suivant l'une ou plusieurs des revendications 1 à 3, caractérisé en ce que la tige recourbée (5) au moins au nombre de une est recourbée dans le sens de la rotation en direction de son extrémité (6) qui plonge dans le liquide (7).

5. Eliminateur de mousse suivant une ou plusieurs des revendications 1 à 4, caractérisé en ce que l'extrémité (6) plongeant dans le liquide (7) est courbée en direction d'une ligne concentrique à l'arbre (1) du rotor.

6. Eliminateur de mousse suivant une ou plusieurs des revendications 2 à 5, caractérisé en ce que la section ouverte de la tige creuse au moins au nombre de une est réglable.

7. Eliminateur de mousse suivant une ou plusieurs des revendications 2 à 6, caractérisé en ce que les sections ouvertes sont différentes aux deux extrémités (6, 8) de la tige creuse au moins au nombre de une.

8. Eliminateur de mousse suivant une ou plusieurs des revendications 1 à 7, caractérisé en ce qu'au moins deux tiges recourbées (5a, 5b) sont montées sur l'arbre (1) du rotor avec une symétrie radiale.

9. Eliminateur de mousse suivant une ou plusieurs des revendications 1 à 8, caractérisé par plusieurs tiges recourbées (5a, 5b) à différents écartements axiaux sur l'arbre (1) du rotor.

10. Eliminateur de mousse suivant une ou plusieurs des revendications 1 à 9, caractérisé par plusieurs tiges recourbées (5) à différentes hauteurs sur l'arbre (1) du rotor.

11. Eliminateur de mousse suivant une ou plusieurs des revendications 1 à 10, caractérisé par un support (4) servant à la fixation de la tige recourbée (5, 5a, 5b), au moins au nombre de une, sur l'arbre (1) du rotor, qui comprend une barre support (1), une vis de blocage (42) et des étriers de fixation (44, 44a, 44b) munis de vis de fixation (45, 45a, 45b) ainsi que de vis entretoises (43, 43a, 43b).

Fig. 2a

Fig. 1a

Fig. 2b

Fig. 1b

# FIG.3